# EUROPEAN PATENT APPLICATION

(11) **EP 4 744 629 A1**
(43) Date of publication of application: **20.05.2026**
(21) Application number: 24944949.7
(22) Date of filing: 16.10.2024
(51) Int. Cl.: A61F 2/38

(54) **BIOLOGICAL TROCHLEAR GROOVE REPLACEMENT ASSEMBLY AND METHOD FOR USING SAME**

(30) Priority: 26.09.2024 CN 202411349761
(71) Applicant: Hefei Longshore Tech Co., Ltd, Hefei, Anhui 230088 (CN)
(72) Inventor: ZHANG, Lifeng, Anhui 230088 (CN)
(74) Representative: Patentgruppen A/S
(86) International application number: PCT/CN2024/125188
(87) International publication number: WO 2026/065604

(57) **Abstract**

The present invention pertains to the field of medical devices, and in particular relates to a biological patellar groove replacement assembly and an operation method. The biological patellar groove replacement assembly includes a prosthesis, a trial implant and a base block. The trial implant has an upper surface generally coinciding and height consistent with those of the prosthesis and can be used to simulate a mounting orientation of the prosthesis. Tuning holes in the trial implant can be used to preliminarily position the trial implant and facilitate tuning of its orientation. Drilling shafts can be inserted into locating holes in the trial implant and first and second locating holes in the base block and then used to drill first and second mounting holes in an osteotomy surface of a femur, enabling accurate positioning of the base block and the prosthesis. In this way, a positional match between the mounting holes in the osteotomy surface of the femur and mounting pegs on the prosthesis can be ensured, which in turn better ensures that the prosthesis can be mounted at an orientation determined by the trial implant, effectively improving the success of a procedure for implanting the prosthesis.

## Description

### Field of the Invention

The present invention pertains to the field of medical devices, and in particular relates to a biological patellar groove replacement assembly and an operation method.

### Description of the Prior Art

Patellar groove replacement (PGR) can be used for treatment of patellar dislocations in pet dogs and cats caused by trochlear injuries, developmental deformities or other reasons. A PGR procedure involves an osteotomy to remove the damaged femoral groove and implantation of a replacement prosthesis. At present, commonly used patellar groove replacement prostheses include a base plate and a trochlear prosthesis, with a press fit being formed between their adjacent surfaces. In a patellar groove replacement procedure, after the position of the trochlear prosthesis is determined, screws are first inserted through the base plate and fix it to a distal end portion of the femur, and the trochlear prosthesis is then press-fitted and secured to the base plate. In this way, not only a smooth, continuous trochlear surface but also an effective joint of the prosthesis assembly with the femur can be obtained. However, in practical applications, loosening, warpage or even separation of the trochlear prosthesis from the base plate may occur, shortening the service life of the prosthesis and necessitating re-surgical repair.

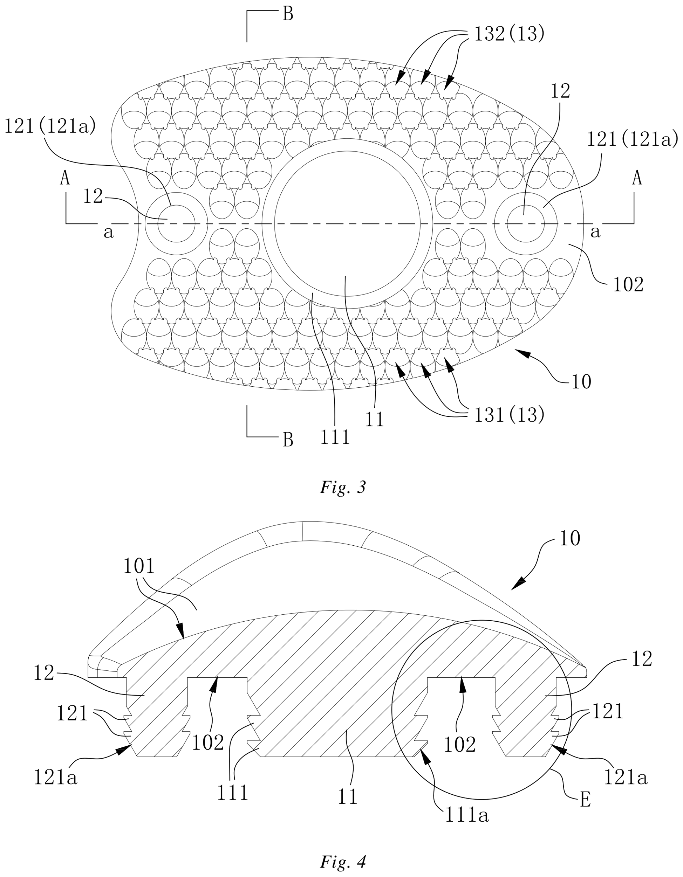

In order to overcome the disadvantages of conventional patellar groove replacement prosthesis assemblies, Chinese Patent. No. CN217723822U discloses a trochlear replacement system comprising a trochlear prosthesis with at least one first fixation member mounted at a distal end of the trochlear prosthesis and at least one second fixation member mounted at the distal end of the trochlear prosthesis. The first fixation member is oriented perpendicularly to a distal end face of the trochlear prosthesis, and the second fixation member is inclined with respect to the distal end face of the trochlear prosthesis. Both the first and second fixation members are proximally joined to the distal end of the trochlear prosthesis and distally inserted into the bone to be subjected to trochlear replacement. Specifically, as shown in Fig. 3 of the patent, two second mounting holes may extend from sides faces of the trochlear prosthesis to the distal end face thereof so as to be oriented at an angle with respect to each other. Accordingly, two second fixation members may be inserted from the opposite sides through the respective second mounting holes obliquely into the femur, restricting the trochlear prosthesis from moving outwardly along an axis of the first fixation member. Although this design entails a trochlear replacement system with enhanced anti-dislodge capability, limited to the size of the trochlear prosthesis, the openings of the two second mounting holes tend to be close to each other on said end face of the trochlear prosthesis. Consequently, the portions of the two second fixation members that are inserted in the femur may be too closely adjacent to each other, or may even interfere with each other. In these situations, reliable anchoring of the second fixation members to the femur cannot be obtained, or the portion of the femur between the two second fixation members cannot heal well, or may become necrotic and eventually fall off, affecting the service life of the trochlear replacement system.

### Summary of the Invention

It is an object of the present invention to provide a biological patellar groove replacement assembly and an operation method, which enable stable and reliable implantation of a trochlear prosthesis.

To this end, the present invention provides:
a biological patellar groove replacement assembly comprising a prosthesis, a trial implant and a base block,
an upper surface of the prosthesis being a continuous curved surface defining a patellar groove, a lower surface of the prosthesis being generally horizontally oriented, the lower surface of the prosthesis provided thereon with a centrally arranged first mounting peg and second mounting pegs arranged near an outer peripheral edge of the prosthesis, cylindrical bodies of the first mounting peg and the second mounting pegs extending from the lower surface of the prosthesis away from the upper surface of the prosthesis,
an upper surface of the trial implant having a contour generally coinciding with that of the upper surface of the prosthesis, a lower surface of the trial implant being generally horizontally oriented, the upper surface of the trial implant and the lower surface of the trial implant having a maximum height difference consistent with a maximum height difference of the upper surface of the prosthesis and the lower surface of the prosthesis, the trial implant defining locating holes extending through the upper surface of the trial implant and the lower surface of the trial implant and tuning holes extending through side wall surfaces of the trial implant and the lower surface of the trial implant, cores of the locating holes having orientations at the lower surface of the trial implant coinciding with orientations of cores of the second mounting pegs at the lower surface of the prosthesis,
the base block generally resembling a plate, the base block defining a first locating hole and second locating holes, all extending through an upper surface of the base block and a lower surface of the base block, a core of the first locating hole having an orientation at the lower surface of the base block coinciding with an orientation of a core of the first mounting peg at the lower surface of the prosthesis, cores of the second locating hole having orientations at the lower surface of the base block coinciding with orientations of cores of the second mounting pegs at the lower surface of the prosthesis.

The present invention also provides an operation method of the biological patellar groove replacement assembly as defined above, comprising the steps of:
A. with the lower surface of the trial implant fitting against an osteotomy surface of a femur, applying pointed shafts through the tuning holes and inserting them into the osteotomy surface of the femur, thereby preliminarily positioning the trial implant;
B. determining whether a lengthwise direction and tilt angle of a patellar groove of the upper surface of the trial implant are consistent with a designed patellar track and, if so, proceeding to step C, or otherwise, removing the pointed shafts, tuning an orientation of the trial implant and looping back to step A;
C. applying second drilling shafts through the locating holes in the trial implant and drilling second mounting holes in the osteotomy surface of the femur;
D. with the second drilling shafts being retained engaged with the osteotomy surface of the femur, pulling off the pointed shafts, separating the trial implant from the osteotomy surface of the femur and then guiding it out along shaft bodies of the second drilling shafts;
E. with the second drilling shafts being inserted into the second locating holes of the base block, guiding the base block down along the shaft bodies of the second drilling shafts until it fits against the osteotomy surface of the femur, applying a first drilling shaft through the first locating hole of the base block and drilling a first mounting hole in the osteotomy surface of the femur;
F. separating the base plate from the osteotomy surface of the femur, guiding it out along the shaft bodies of the first and second drilling shafts and removing the first and second drilling shafts; and
G. with the first mounting peg of the prosthesis abutting against an opening edge of the first mounting hole and the second mounting pegs of the prosthesis abutting against opening edges of the second mounting holes, securely press-fitting the prosthesis onto the osteotomy surface of the femur so that the lower surface of the prosthesis fits against the osteotomy surface of the femur, completing implantation of the prosthesis.

The present invention offers the following benefits over the prior art: in the biological patellar groove replacement assembly, the trial implant has an upper surface generally coinciding and height consistent with those of the prosthesis and can be used to simulate a mounting orientation of the prosthesis. The tuning holes in the trial implant can be used to preliminarily position the trial implant and facilitate tuning of its orientation. Drilling shafts can be inserted into the locating holes in the trial implant and the first and second locating holes in the base block and then used to drill first and second mounting holes in an osteotomy surface of a femur, enabling accurate positioning of the base block and the prosthesis. In this way, a positional match between the mounting holes in the osteotomy surface of the femur and the mounting pegs on the prosthesis can be ensured, which in turn better ensures that the prosthesis can be mounted at an orientation determined by the trial implant, effectively improving the success of a procedure for implanting the prosthesis.

### Brief Description of the Drawings

The drawings accompanying this specification, as well as reference numerals therein, are described briefly below, in which:
Figs. 1 and 2 are schematic perspective views of a prosthesis according to a first embodiment;
Fig. 3 is a bottom view of the prosthesis according to the first embodiment;
Fig. 4 is a cross-sectional view taken along A-A of Fig. 3;
Fig. 5 is a schematic enlarged view of part E of Fig. 4;
Fig. 6 is a cross-sectional view taken along B-B of Fig. 3;
Fig. 7 is a schematic perspective view of a prosthesis according to a second embodiment;
Fig. 8 is a bottom view of the prosthesis according to the second embodiment;
Fig. 9 is a cross-sectional view taken along C-C of Fig. 8;
Fig. 10 is a schematic enlarged view part F of Fig. 9;
Fig. 11 is a cross-sectional view taken along D-D of Fig. 8;
Figs. 12 and 13 are schematic perspective views of a trial implant in the first embodiment;
Fig. 14 is a schematic perspective view of one half of the trial implant in the first embodiment;
Fig. 15 is a partially cutaway schematic perspective view of the trial implant in the first embodiment along a plane defined by cores of tuning holes;
Fig. 16 of a top view of a trial implant in the second embodiment;
Fig. 17 is a cross-sectional view taken along G-G of Fig. 16;
Fig. 18 is a schematic perspective view of the trial implant in the second embodiment; and

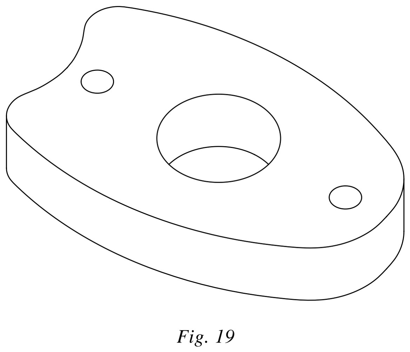

Fig. 19 is a schematic perspective view of a base block in the first embodiment.

### Detailed Description of the Preferred Embodiments

Specific embodiments of the present invention are described in greater detail below, by way of example, with reference to the accompanying drawings.

A biological patellar groove replacement assembly includes a prosthesis 10, a trial implant 20 and a base block 30.

The prosthesis 10 includes an upper surface 101 defining a valley-like profile in a widthwise direction of the prosthesis 10, which is sunken in the middle and raised at both ends, and a ridged profile in a lengthwise direction, which is swollen in the middle and slopes down towards both ends. The upper surface 101 of the prosthesis is a smooth, continuous, curved surface having a sunken central region, which defines a patellar groove in cooperation with a patella. The patella is able to glide within the patellar groove defined by the upper surface 101 of the prosthesis, while ridges of the prosthesis 10 at sides thereof opposing each other in the widthwise direction are able to prevent the patella from falling off from either side. In this way, displacement of the patella can be well guided and restricted. Side surfaces 103 of the prosthesis extend downwardly from the opposing sides of the upper surface 101 of the prosthesis and join with a lower surface 102 of the prosthesis along their bottom edges. The lower surface of the prosthesis is oriented generally horizontally. The prosthesis 10 is provided at the bottom with a central first mounting peg 11 and second mounting pegs 12 near an outer peripheral edge of the prosthesis 10. The first mounting peg 11 and the second mounting pegs 12 are provided to be inserted to a femur, thereby anchoring the prosthesis 10 to the femur.

The trial implant 20 includes an upper surface 201, which overall coincides with the upper surface 101 of the prosthesis. Side wall surfaces 203 of the trial implant extend downwardly from opposing sides of the upper surface 201 of the trial implant and join with a lower surface 202 of the trial implant along their bottom edges. The lower surface 202 of the trial implant is oriented generally horizontally. A maximum height difference between the upper surface 201 of the trial implant and the lower surface 202 of the trial implant is consistent with a maximum height difference between the upper surface 101 of the prosthesis and the lower surface 102 of the prosthesis. That is to say, the trial implant 20 has a height, which is consistent with a height of a main body of the prosthesis 10. Here, the height of the main body of the prosthesis 10 is defined as a height of the prosthesis 10 after the first mounting peg 11 and the second mounting pegs 12 have been removed therefrom. In other words, the trial implant 20 has an outer contour, which overall matches an outer contour of the prosthesis 10 with the first mounting peg 11 and the second mounting pegs 12 being removed.

The upper surface 201 of the trial implant includes a curved surface portion, which coincides with the upper surface 101 of the prosthesis. The trial implant 20 defines locating holes 21 extending through both the upper surface 201 of the trial implant and the lower surface 202 of the trial implant. Orientations of cores of the locating holes 21 with respect to the lower surface 202 of the trial implant coincide with orientations of cores of the second mounting pegs 12 with respect to the lower surface 102 of the prosthesis. Here, the coincidence of the orientations means that the locating holes 21 may be through holes drilled using drilling shafts, in and with which the second mounting pegs 12 can be inserted and reliably engaged. As can be obviously seen, on the upper surface 201 of the trial implant, recessed defects may be formed around top edges of the locating holes 21 that extend through the upper surface 201 of the trial implant and the lower surface 202 of the trial implant, differentiating upper surface portions of the trial implant 20 around the upper edges of the locating holes 21 from corresponding upper surface portions of the prosthesis 10. The upper surface 201 of the trial implant only generally coincides with, but is not exactly consistent with, the upper surface 101 of the prosthesis. Since the height of the trial implant 20 is consistent with the height of the main body of the prosthesis 10, the trial implant 20 can be positioned on an osteotomy surface of the femur so that its upper surface 201 of the trial implant has the same orientation as the upper surface 101 of the prosthesis in a mounted state of the prosthesis 10, in which the lower surface 102 of the prosthesis fits against the osteotomy surface of the femur. Tuning holes 22 extend through the side wall surfaces 203 of the trial implant and the lower surface 202 of the trial implant. Pointed shafts can be inserted through the tuning holes 22 to preliminarily position the trial implant 20 on the osteotomy surface of the femur.

The base block 30 is generally a plate-like block and defines a first locating hole 31 and second locating holes 32, all extending through an upper surface 301 of the base block and a lower surface 302 of the base block. An orientation of a core of the first locating hole 31 with respect to the lower surface 302 of the base block coincides with an orientation of a core of the first mounting peg 11 with respect to the lower surface 102 of the prosthesis. Orientations of cores of the second locating holes 32 with respect to the lower surface 302 of the base block coincide with the orientations of the cores of the second mounting pegs 12 with respect to the lower surface 102 of the prosthesis. Here, the coincidence of the orientations means that the first locating hole 31 may be a through hole drilled using a drilling shaft, in and with which the first mounting peg 11 can be inserted and engaged, and that the second locating holes 32 may be through holes drilled using drilling shafts, in and with which the second mounting pegs 12 can be inserted and engaged.

An operation method of the biological patellar groove replacement assembly as discussed above specifically includes the steps as described below.

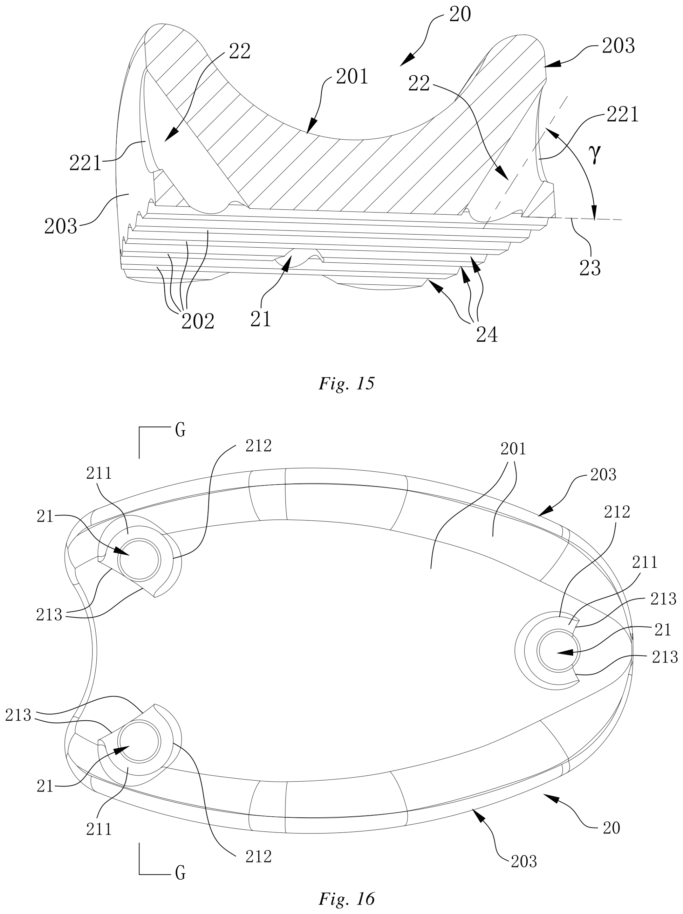

A. With the lower surface 202 of the trial implant fitting against the osteotomy surface of the femur, the pointed shafts are inserted through the tuning holes 22 into the osteotomy surface of the femur, thereby preliminarily positioning the trial implant 20. As shown in Fig. 15, the tuning holes 22 are oblique, allowing the pointed shafts to be obliquely inserted into cancellous bone at a central area of the osteotomy surface of the femur. This can be accomplished even manually by a surgeon, and the trial implant 20 can be attached and detached simply by inserting and removing the pointed shafts. Therefore, an orientation of the trial implant 20 on the osteotomy surface of the femur can be easily tuned.

B. A determination is made of whether a lengthwise direction and tilt angle of the patellar groove of the upper surface 201 of the trial implant are consistent with a designed patellar track. If so, the operation method proceeds to step C. Otherwise, the pointed shafts are removed, and the operation method loops back to step A to tune the orientation of the trial implant 20. A patella or patellar prosthesis may be tried on the preliminarily positioned trial implant 20 to allow a determination to be made intuitively of whether the orientation of the trial implant 20 is as desired by the design. Since the outer contour of the trial implant 20 overall matches that of the prosthesis 10, once the orientation of the trial implant 20 is determined, a mounting orientation of the prosthesis 10 can be accordingly determined.

C. Second drilling shafts are inserted through the locating holes 21 in the trial implant 20 to drill second mounting holes in the osteotomy surface of the femur. In this step, the pointed shafts remain inserted to ensure that the trial implant 20 is oriented as designed. After forming the holes, the second drilling shafts are retained on the femur and serve to provide guidance to the subsequent surgical operations. As a result of this step, the second mounting holes are formed in the osteotomy surface of the femur in alignment with the corresponding second mounting pegs 12 of the prosthesis 10.

D. With the second drilling shafts being retained on the osteotomy surface of the femur, the pointed shafts are pulled off, and the trial implant 20 is separated from the osteotomy surface of the femur and then removed along the second drilling shafts. The second drilling shafts remain engaged with the locating holes 21 in the trial implant 20 to enable positioning of the trial implant 20 and the base block 30. Therefore, the pointed shafts can be removed, avoiding obscuring a field of view or hindering operations in the procedure. As a result of this step, the trial implant 20 completes its functions of simulating a process to tune the mounting orientation of the prosthesis 10 and helping to drill the second mounting holes in desired positions and orientations.

E. The base block 30 is held so that the second drilling shafts are inserted into the second locating holes 32 and then further moved down along the second drilling shafts into contact with the osteotomy surface of the femur. A first drilling shaft is inserted into the first locating hole 31 of the base block 30 and used to drill a first mounting hole in the osteotomy surface of the femur. In other words, the second drilling shafts are first inserted into the second locating holes 32 of the base block 30, and the base block 30 is then moved down under the guidance of the second drilling shafts into contact with the osteotomy surface of the femur. With the base block 20 being retained in contact with the osteotomy surface of the femur, the first drilling shaft is used to drill the first locating hole 31. As a result of this step, the first mounting hole is formed in the osteotomy surface of the femur in alignment with the corresponding first mounting peg 11 on the prosthesis 10.

F. The base block 30 is separated from the osteotomy surface of the femur and removed along the first and second drilling shafts, followed by removal of the first and second drilling shafts. After step E is completed, the first and second mounting holes for fixing the mounting pegs at the bottom of the prosthesis 10 have been formed in the osteotomy surface of the femur. Therefore, the base block and the drilling shafts can be removed to enable implantation and attachment of the prosthesis 10.

G. The prosthesis 10 is held so that the first mounting peg 11 abuts against an opening edge of the first mounting hole in the osteotomy surface of the femur and that the second mounting pegs 12 abut against opening edges of the second mounting holes in the osteotomy surface of the femur and then press-fitted onto the osteotomy surface of the femur so that the lower surface 102 of the prosthesis fits against the osteotomy surface of the femur, completing the implantation of the prosthesis 10.

Therefore, the trial implant 20 is provided to simulate a process to tune the mounting orientation of the prosthesis 10 and to enable drilling of the second mounting holes in desired positions and orientations, and the base block 30 is provided to enable drilling of the first mounting hole in a desired position and orientation. Thus, once the first and second mounting holes are formed in the osteotomy surface of the femur, the mounting of the prosthesis 10 can be carried out. Since the first mounting peg 11 is provided at the bottom of the prosthesis 10 around the center thereof, if a locating hole were formed in the trial implant 20 in alignment with the first mounting peg, there would be a recessed defect around the center of the upper surface 201 of the trial implant. This defect may affect a determination made as to a degree of matching between the trochlear surface mimicked by the upper surface 201 of the trial implant and a patella, which is crucial to the success of the procedure. For this reason, the locating holes 21 in the trial implant 20 are used only to enable drilling of the second mounting holes in desired positions and orientations, while the base block 30 is added to enable drilling of the first mounting hole in a desired position and orientation with the aid of locators provided by the drilling shafts that have been used to drill the second mounting holes.

In order to ensure that the pegs on the prosthesis 10 have the same orientations as the holes in the trial implant 20 and the base block 30, in a first preferred embodiment, the lower surface 102 of the prosthesis, the lower surface 202 of the trial implant and the lower surface 302 of the base block have the same outer peripheral contour. In this way, these components can be fabricated in a single process, reducing dimensional deviations among them that may be otherwise introduced by different machining tolerances of different processes.

In addition, in order to enable more reliable and stable attachment of the prosthesis 10 to the osteotomy surface of the femur, the prosthesis 10 may be symmetric with respect to a line, which is parallel to a lengthwise direction of the lower surface 102 of the prosthesis and is located at a center of the lower surface 102 of the prosthesis in a widthwise direction thereof. In case of the lower surfaces of the prosthesis 10, the trial implant 20, and the base block 30 having the same outer peripheral contour, the trial implant 20 and the base block 30 may each be symmetric with respect to a line, which is parallel to a lengthwise direction of the specific lower surface and passes through a center of the lower surface in a widthwise direction thereof.

In specific implementations, the osteotomy surface of the femur may form as a result of an osteotomy performed on the trochlear surface. Soft cancellous bone is exposed in a central region of this surface, and hard cortical bone is exposed in the remaining peripheral region. The second mounting pegs 12 are arranged adjacent a peripheral edge of the prosthesis 10 and therefore can be attached to the osteotomy surface of the femur at locations near the cortical bone. This allows the second mounting pegs 12 to be reliably attached to the femur even when they have a relatively small cross-section. On the other hand, in order to ensure stable attachment of a central portion of the prosthesis 10 to the femur, the first mounting peg 11 may have a cross-section, which is large enough to allow a cylindrical body of the first mounting peg 11 to be located adjacent the cortical bone exposed at the osteotomy surface of the femur. With this arrangement, the first mounting peg 11 can be more reliably attached to the femur. In other words, the first mounting peg 11 may have a larger cross-sectional area and outer peripheral contour than the second mounting pegs 12. In order to facilitate fabrication and reduce stimulation to bone tissue that may provoke inflammatory response, in the present embodiment, the first mounting peg 11 and the second mounting pegs 12 are generally circular, and the first mounting peg 21 has a greater outer diameter than the second mounting pegs 22. In other embodiments, the first mounting peg 11 and the second mounting pegs 12 may also be non-circular. For example, they may be polygonal, elliptical or otherwise-shaped, as long as they can be inserted into the osteotomy surface of the femur.

In the first embodiment, as shown in Fig. 3, a size of the first mounting peg 11 in the widthwise direction of the prosthesis 10 is greater than 1/3 of a maximum size of the prosthesis 10 in the same direction. This allows the first mounting peg 11 to be more reliably attached to the femur, but may create difficulties in forming the second mounting pegs 12 at the bottom of the prosthesis 10 on opposite sides of the first mounting peg 11. For this reason, the second mounting pegs 12 may be arranged on two ends of prosthesis 10, which oppose each other in the lengthwise direction thereof. That is, the second mounting pegs 12 may be arranged on front and rear ends of the prosthesis 10. Accordingly, in order to enable stable attachment of the prosthesis 10 to the femur, at least two second mounting pegs 12 are needed.

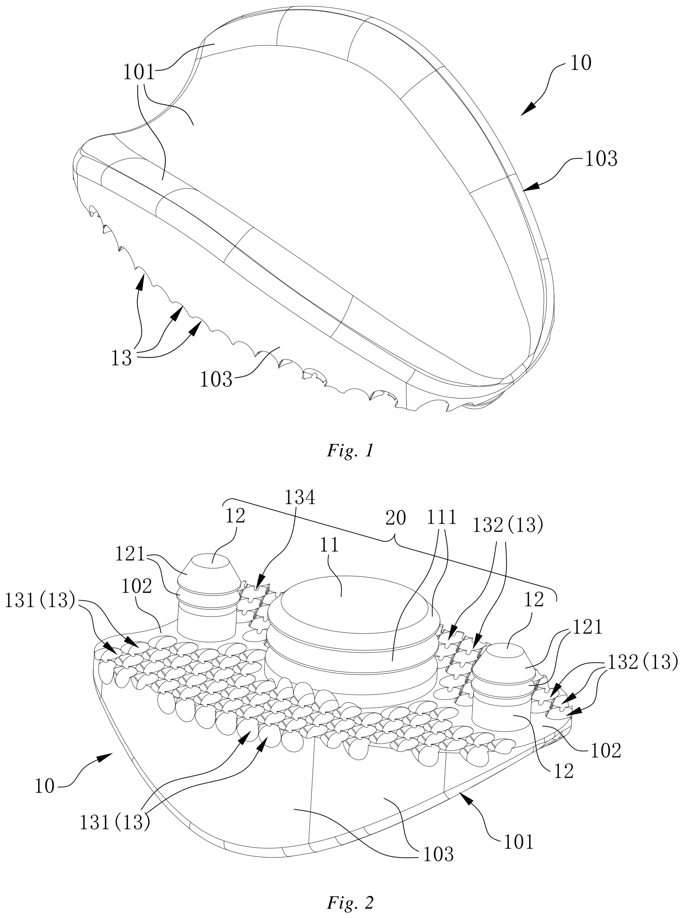

In order to enable immediate stability of the prosthesis 10 as soon as it is implanted, in the first embodiment, as shown in Figs. 2 and 4, the first mounting peg 11 has a depending end defining a first retention collar 111, and each second mounting peg 12 also has a depending end defining a second retention collar 121. The first retention collar 111 and the second retention collars 121 each have a collar surface proximate the horizontally oriented lower surface 102 of the prosthesis. When the first mounting peg 11 and the second mounting pegs 12 are inserted in the corresponding mounting holes, bone tissue will be embedded in grooves between the retention collars and cylindrical bodies of the mounting pegs. The bone tissue in the grooves will abut against the collar surfaces of the first retention collar 111 and the second retention collars 121 along the cores of the mounting pegs, blocking the first mounting peg 11 and the second mounting pegs 12 from outward dislodgement and imparting immediate stability to the prosthesis 10. Over a period of time after prosthesis 10 is implanted, the bone tissue will grow and fill the grooves between the retention collars and the cylindrical bodies of the mounting pegs, further ensuring long-term stability of the implanted prosthesis.

Further, the first mounting hole drilled in the osteotomy surface of the femur is desired to have a smaller diameter than an outer contour of the first retention collar 111, and the second mounting holes drilled in the osteotomy surface of the femur are desired to have a smaller diameter than outer contours of the second retention collars 121. Accordingly, the locating holes 21 and the second locating holes 32 has a diameter that is smaller than or equal to a maximum outer diameter of the second mounting pegs 12, and the first locating hole 31 has a diameter that is smaller than or equal to a maximum outer diameter of the first mounting peg 11.

The second mounting pegs 12 arranged near the outer peripheral edge of the prosthesis 10 can be more easily observed than the first mounting peg 11 and therefore provide guidance to implanting and mounting operations, which increases positioning accuracy of the implanted prosthesis 10.

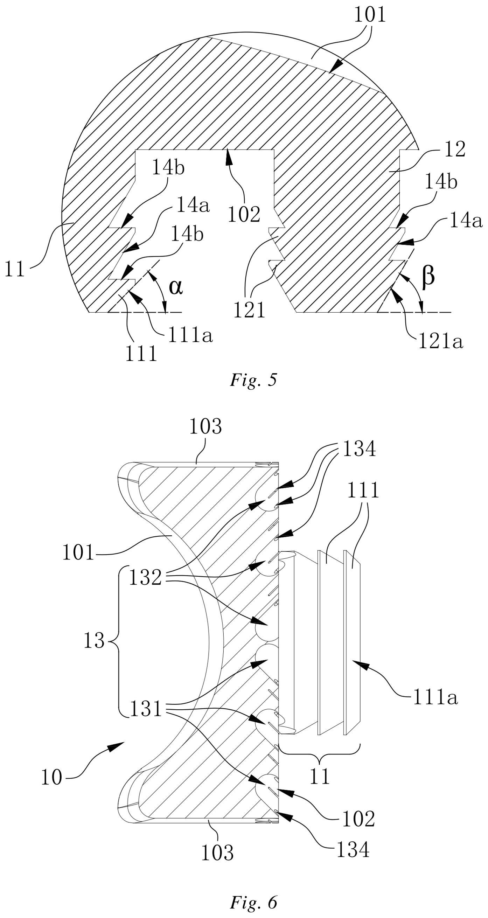

In the first embodiment, as shown in Fig. 4, depending end faces of the first mounting peg 11 and the second mounting pegs 12 are spaced at the same distance from the lower surface 102 of the prosthesis. That is, the first mounting peg 11 and the second mounting pegs 12 project the same distance. In addition, the first retention collar 111 and the second retention collars 112 are tapered. The first retention collar 111 is adjacent the depending end face of the first mounting peg 11, and a first tapered collar surface 111a of the first retention collar 111 joins the depending end face of the first mounting peg 11. The second retention collars 121 are adjacent the depending end faces of the second mounting pegs 12, and second tapered collar surfaces 121a of the second retention collars 121 join the depending end faces of the second mounting pegs 12. A degree of taper of the first tapered collar surface 111a is greater than or equal to a degree of taper of the second tapered collar surfaces 121a. That is, ∠α < ∠β, as shown in Fig. 5. With this arrangement, when the prosthesis 10 is pressed down in the orientation shown in Fig. 4, the second mounting pegs 12 will be more easily pressed into the corresponding holes in the femur than the first mounting peg 11, providing guidance to the attachment of the first mounting peg 11. In the present embodiment, the cores of the three mounting pegs are located in a single plane, which is perpendicular to the lower surface 102 of the prosthesis and parallel to the lengthwise direction of the prosthesis 10. With this arrangement, when the prosthesis 10 is being pressed down, its stability in the lengthwise direction can be ensured. Once the prosthesis 10 is pressed down in place, the lower surface 102 of the prosthesis will fit against the osteotomy surface of the femur, restricting the prosthesis 10 from pivoting toward either side in the widthwise direction. Effective positioning of the prosthesis 10 is achieved.

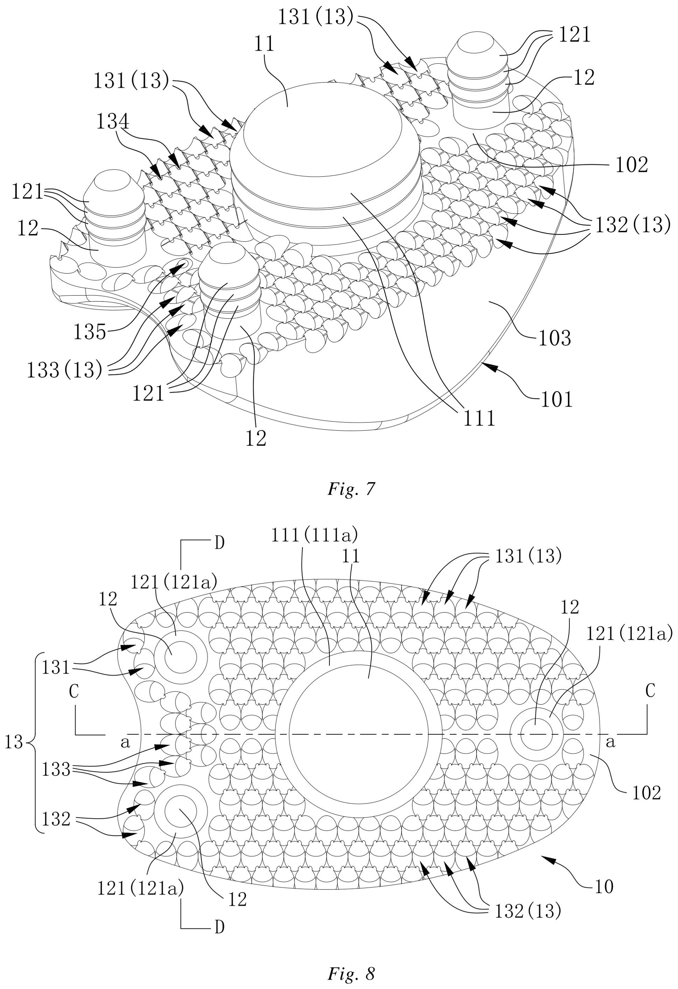

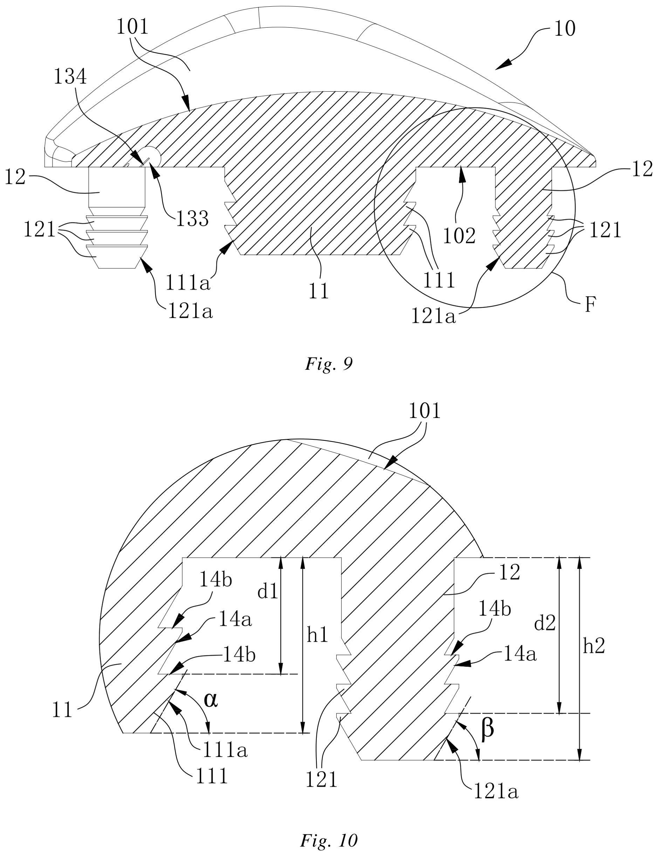

In a second embodiment, as shown in Fig. 9, the depending end faces of the second mounting pegs 12 is spaced at a greater distance than the first mounting peg 11 from the lower surface 102 of the prosthesis. That is, h2 > h1, as shown in Fig. 10. In other words, the second mounting pegs 12 project a greater distance than the first mounting peg 11. With this arrangement, when the prosthesis 10 is pressed down in the orientation shown in Fig. 9, the second mounting pegs 12 will be pressed into the corresponding holes in the femur earlier than the first mounting peg 11. After the second mounting pegs 12 besides the first mounting peg 11 are pressed into the corresponding holes in the femur, the prosthesis 10 can be further pressed down in the same orientation to ensure reliable attachment of the first mounting peg 11 to the femur. Moreover, in the present embodiment, a maximum distance from the collar surfaces of the second retention collars 121 to the lower surface 102 of the prosthesis is greater than a maximum distance from the collar surface of the first retention collar 11 to the lower surface 102 of the prosthesis. That is, d2 > d1, as shown in Fig. 10. With this arrangement, once the second retention collars 121 adjacent the depending end faces of the second mounting pegs 12 are received in the mounting holes in the femur, positioning of the prosthesis 10 can be carried out. In the present embodiment, the degree of taper of the first tapered collar surface 111a is equal to the degree of taper of the second tapered collar surfaces 121a of the second retention collars 232. That is, ∠α = ∠β, as shown in Fig. 10. Specifically, in the present embodiment, there are three second mounting pegs 12, which have the same columnar shape and project the same distance. The three second mounting pegs 12 are arranged in symmetry with respect to an axis of symmetry a, as shown in Fig. 8. With this arrangement, when the prosthesis 10 is being pressed down, its orientation can be effectively kept from tilting at large angles.

In the first and second embodiments, each of the first retention collar 111 and the second retention collars 121 is a frustum having a tapered surface 14a, which flares from the depending end of the respective mounting peg toward the lower surface 102 of the prosthesis, and an annular collar surface 14b, which joins the tapered surface 14a and the cylindrical body of the mounting peg. That is, each of the first retention collar 111 and the second retention collars 121 includes a tapered surface 14a defining a smaller diameter at the bottom and a larger diameter at the top. An upper edge of the tapered surface 14a is joined by a horizontally oriented collar surface 14b, a cylindrical surface of the corresponding mounting peg, or a tapered surface 14a of another retention collar that is located above. In other embodiments, the first retention collar 111 and the second retention collars 121 may also be annular collars with parallel upper and lower collar surfaces, or bands of bumps or ribs surrounding the cylindrical bodies of the mounting pegs. Regardless of what form they take, their collar surfaces adjacent the lower surface 102 of the prosthesis can work together with bone tissue to block the mounting pegs from dislodging outwardly along their cores from the mounting holes in the femur.

In the first and second embodiments, in order to enhance anti-dislodge capability of the prosthesis 10, as shown in Figs. 2 and 7, the depending end of the first mounting peg 11 defines at least two first retention collars 111, and at least two second retention collars 121 are provided on the cylindrical body of each second mounting peg 12. In other embodiments, one, two or more first retention collars 111 and second retention collars 121 may be provided, as required.

In the first and second embodiments, two and more than two mounting pegs are provided at the bottom of the prosthesis 10, respectively. In order to facilitate the implantation, the cores of the first mounting peg 11 and the second mounting pegs 12 are arranged perpendicularly to the lower surface 102 of the prosthesis.

In order to ensure long-term stability of the trochlear prosthesis, as shown in Figs. 2, 3 and 6, the lower surface 102 of the prosthesis defines blind osseointegration holes 13 for growth of bone tissue therein. Therefore, the osseointegration holes 13 are spaced apart from the upper surface 101 of the prosthesis. This ensures that the upper surface 101 of the prosthesis is a continuous, smooth, curved surface, which can stably and reliably provide support to a patella and guide its gliding. In the first embodiment, the osseointegration holes 13 are obliquely open downwardly with respect to the prosthesis 10. That is, the osseointegration holes 13 are bottomed toward the center of the upper surface 101 of the prosthesis and open away from the center of the upper surface 101 of the prosthesis. Specifically, as shown in Fig. 6, core lines of the osseointegration holes 13 intersect the lower surface 102 of the prosthesis. Moreover, the core lines of the osseointegration holes 13 intersect a line, which is perpendicular to the lower surface 102 of the prosthesis and passes through its center, above the lower surface 102 of the prosthesis. Alternatively, the core line of each osseointegration hole 13 is not located in the same plane as the line that is perpendicular to the lower surface 102 of the prosthesis and passes through its center, and a line perpendicular to both these lines is located above the lower surface 102 of the prosthesis. After the prosthesis 10 is implanted, bone tissue can grow into the osseointegration holes 13, achieving biological fixation that ensures long-term stability of the implanted prosthesis. The oblique osseointegration holes 13, when compared with conventional ones that are perpendicular to the lower surface 102 of the prosthesis, allow bone tissue to obliquely grow upwards into the osseointegration holes 13 and adhere to inner walls of the osseointegration holes 13, effectively restricting the prosthesis 10 from displacement away from the osteotomy surface of the femur and thereby avoiding post-implantation loosening or falling off of the prosthesis 10 after a long time of use.

In the first embodiment, as shown in Fig. 6, the osseointegration holes 13 include first holes 131 and second holes 132. Core lines of the first holes 131 and the second holes 132 intersect above the lower surface 102 of the prosthesis. Alternatively, the core lines of the first holes 131 and the second holes 132 are not located in the same planes, and lines perpendicular to both these lines are located above the lower surface 102 of the prosthesis. Herein, the terms "above" and "below" are used with respect to the orientation of Figs. 4 and 9. Specifically, "above" and "below" correspond to the left and right side of Figs. 4 and 9, respectively.

Specifically, as shown in Fig. 6, the first holes 131 and the second holes 132 are oriented at an angle with respect to each other. With this arrangement, bone tissue that has grown into the crossing holes locks the prosthesis 10, ensuring tight attachment of the lower surface 102 of the prosthesis to the osteotomy surface of the femur. In this embodiment, the osseointegration holes 13 are scattered over the entire lower surface 102 of the prosthesis, maximizing ingrowth of bone tissue and tightening the attachment to the prosthesis 10. For each of fabrication, the first holes 131 and the second holes 132 are formed on opposite sides of the aforementioned axis of symmetry of the prosthesis. With this arrangement, the first holes 131 and the second holes 132 can be formed on the opposite sides of the lower surface 102 of the prosthesis within a single fabrication process. Moreover, in this embodiment, the cores of the first holes 131 are arranged in parallel, and the first holes 131 form a first set of holes. Likewise, the cores of the second holes 132 are arranged in parallel, and the second holes 132 form a second set of holes. The first and second sets of holes are formed on the opposite sides of the lower surface 102 of the prosthesis. With this arrangement, the first holes 131 and the second holes 132 can be formed using spaced sets of drill bits each consisting of parallel drill bits and operating on the opposite sides of the lower surface 102 of the prosthesis, allowing all the osseointegration holes 13 to be formed in a single pass.

Further, in the first embodiment, adjacent osseointegration holes 13 in each set of holes are brought into communication. Specifically, as shown in Figs. 2 and 6, each adjacent pair of osseointegration holes 13 with parallel cores are brought in communication at a notch 134 formed in their adjacent walls. With this arrangement, newly grown bone tissue can be interconnected into a one-piece unitary structure, which is even more tightly attached to the prosthesis 10. In other embodiments, each adjacent pair of osseointegration holes 13 with non-parallel cores may be brought into communication at their bottom.

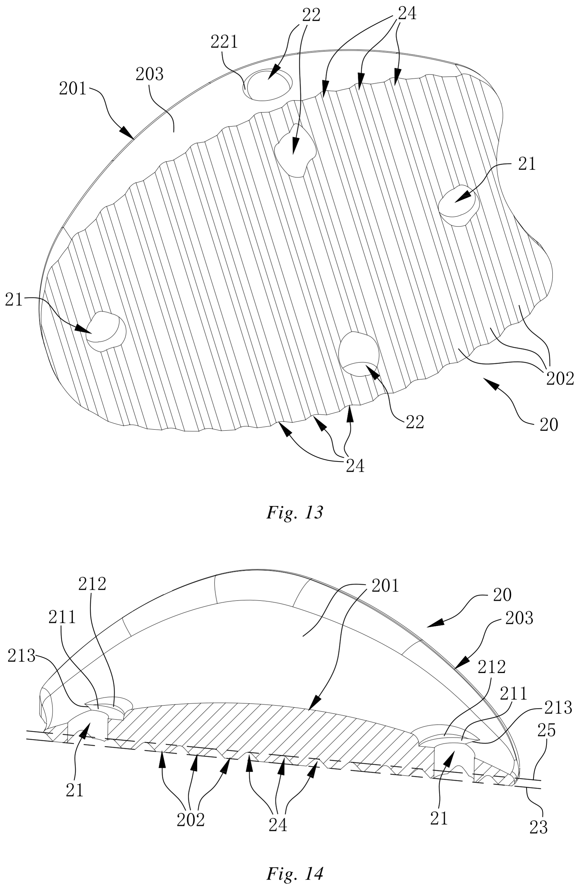

In the second embodiment, as shown in Figs. 7, 9 and 11, two second mounting pegs 22 are spaced apart and both provided at the rear end of the prosthesis 10. Blocked by the cylindrical bodies of these second mounting pegs 22, it is difficult to form first holes 131 and second holes 132 on the lower surface 102 of the prosthesis between the two second mounting pegs 22 provided at the rear end of the prosthesis 10. For this reason, in the second embodiment, the osseointegration holes 13 further include third holes 33 with core lines intersecting planes containing the core lines of the first holes 131 or the second holes 132. With this arrangement, more osseointegration holes 33 can be formed on the lower surface 102 of the prosthesis, and bone tissue can further grow into the third holes 33, additionally restricting displacement of the prosthesis 10 with respect to the osteotomy surface of the femur. Similarly, for each of fabrication, the third holes 33 have parallel cores and form a third set of holes. As in the first embodiment, the osseointegration holes 13 are also scattered over the entire lower surface 102 of the prosthesis in this embodiment. Specifically, as shown in Fig. 13, the first holes 131 and the second holes 132 are arranged on opposite sides of the axis of symmetry of the prosthesis, and the third holes 33 are spaced apart and arranged in symmetry between the first mounting peg 11 and the second mounting pegs 12 on the opposite sides of the axis of symmetry of the prosthesis. The core lines of the third holes 33 are perpendicular to the planes containing the core lines of the first holes 131 or the second holes 132. Differing from the first embodiment, the third holes 33 are brought into communication at the bottom with adjacent first holes 131 or second holes 132 in this embodiment, forming through bores 135, as shown in Fig. 7. Bone tissue can grow into the through bore 135 into an interconnected one-piece structure.

Referring to Fig. 7, in the second embodiment, the osseointegration holes 13 are scattered on the opposite sides of the prosthesis 10, and there are osseointegration holes 13 around the bottom edges of the side wall surfaces 103 of the prosthesis, or around the joints of the side wall surfaces 103 of the prosthesis and the lower surface 102 of the prosthesis. With this arrangement, bone tissue can grow upwards and adhere to the bottom edges of the side wall surfaces 103 of the prosthesis, effectively restricting displacement of the prosthesis 10 in its widthwise direction.

Preferably, the osseointegration holes 13 are spaced at their bottom from the lower surface 102 of the prosthesis at a distance of less than 2 mm. This allows as much as bone tissue to grow into the prosthesis 10 and ensures stability of the prosthesis 10 at its bottom, thereby ensuring long-term post-implantation reliability of the trochlear prosthesis.

The trochlear prosthesis 10 is implanted so that the first mounting peg 11 and the second mounting pegs 12 at the bottom of the prosthesis 10 pass through the osteotomy surface of the femur into the femur, with the lower surface 102 of the prosthesis fitting against the osteotomy surface of the femur. Therefore, a projecting height of the implanted trochlear prosthesis 10 from the osteotomy surface of the femur is determined by a maximum distance between the upper surface 101 of the prosthesis and the lower surface 102 of the prosthesis. Compared with commonly used existing assembly products, a base plate is omitted from the inventive prosthesis 10, so it has a lower profile above the osteotomy surface of the femur when surgically implanted. That is, the replacement prosthesis has a reduced footprint within an animal's articular capsule, which can not only facilitate a surgeon's suturing operation within the articular capsule, but also leave more space in the articular capsule for smoother, unimpeded movement of a patella therein. Compared with the trochlear replacement system disclosed in Chinese Patent. No. CN217723822U, adding the first mounting peg 11, second mounting pegs 12 and osseointegration holes 13 to the bottom of the prosthesis 10 of the present invention dispenses with the use of a separate base plate or securing members while allowing the prosthesis to have a small projecting height after it is implanted, which ensures sufficient space of movement within an articular capsule. Therefore, after being implanted, the trochlear prosthesis enables flexible joint movement while providing improved reliability and stability.

In order to prevent bacterial contamination or other accidents that may occur to the trial implant 20 and the base block 30 due to slippage of the trial implant 20 during any adjustment made to its orientation, or of the trial implant 20 and the base block 30 off a surgeon's hand during a surgical procedure, the lower surface 202 of the trial implant, the upper surface 301 of the base block and the lower surface 302 of the base block may each have an anti-slip texture.

As shown in Fig. 14, in order to ensure that the trial implant 20 has a height consistent with the height of the main body of the prosthesis 10, valleys of the anti-slip texture of the lower surface 202 of the trial implant are desired to be located in a single planar base surface 23. That is, the base surface 23 serves a basis for measuring the height of the trial implant 20. Further, in order to be consistent with the first mounting peg 11, whose core is perpendicular to the lower surface 102 of the prosthesis, the core lines of the locating holes 21 may also be perpendicular to the base surface 23, and the locating hole 21 may be provided on the front or rear end of the trial implant 20.

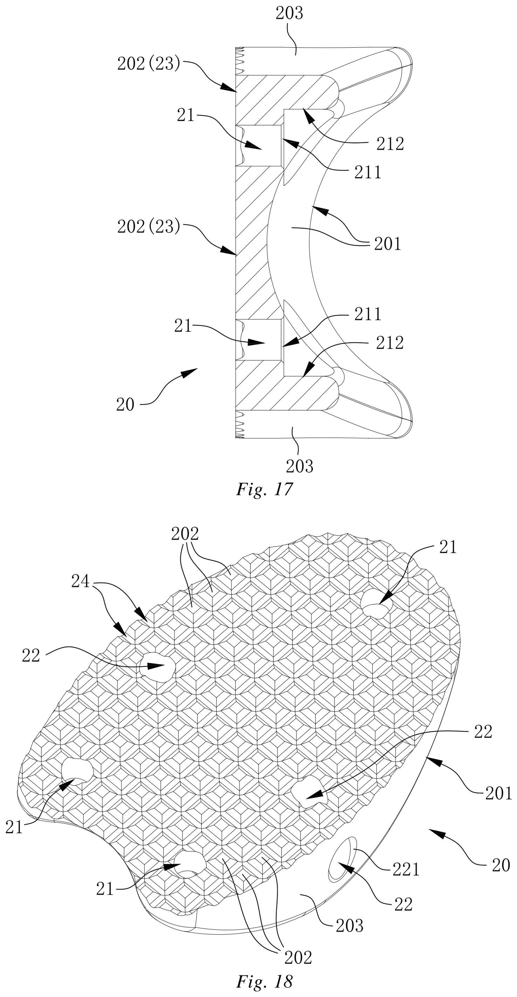

The anti-slip texture may consist of groove or cavities on the base surface 23. As shown in Fig. 14, in the first embodiment, base peaks of the anti-slip texture are also located in a single peak plane 25. The base surface 23 is parallel to the peak plane 25. After the prosthesis 10 is implanted, a patella can move in the patellar groove defined by the upper surface 101 of the prosthesis in the lengthwise direction thereof. During an orientation assessment of the upper surface 201 of the trial implant, in order to prevent the trial implant 20 glide in its lengthwise direction relative to the osteotomy surface of the femur as a patella or patella prosthesis moves in the patellar groove defined by the upper surface 101 of the prosthesis in the lengthwise direction thereof, in preferred embodiments, trial implant grooves 24 are provided on the base surface 23, which extend generally in the widthwise direction of the trial implant 20. In the first embodiment, as shown in Figs. 14 and 15, trial implant grooves 24 are provided on the base surface 23, which extend in the widthwise direction of the trial implant 20 and are spaced apart at equal intervals in the lengthwise direction of the trial implant 20, forming a texture with a striped pattern. In the second embodiment, as shown in Fig. 18, grooves extending in the widthwise direction of the trial implant 20 and grooves extending in the lengthwise direction thereof are provided on the base surface 23, which together form an anti-slip texture with a chessboard-shaped pattern. In other embodiments, the anti-slip textures of the trial implant 20 and the base block 30 may also consist of wavy grooves extending generally in the widthwise direction of the trial implant 20 or the base block 30, or have a pattern resembling a honeycomb, a grid or the like, as long as slippage of the trial implant 20 and the base block 30 can be prevented.

As shown in Fig. 15, in order to prevent the needles inserted in the locating holes 21 from interfering with each other, core lines of the tuning holes 22 form an acute angle γ with the base surface 23. Lower opening ends of the two tuning holes 22 on the opposite sides of the trial implant 20 are spaced at a distance greater than 1/2 of a size of the trial implant 20 measured along a line connecting the locations. With this arrangement, even when inserted in the osteotomy surface of the femur, the needles are still spaced apart without mutual interference. This can not only prevent mutual interference of the needles, but also allows each needle to be tuned in a certain range. In order to facilitate observation of the needles while they are being tuned, the tuning holes 22 may be flared outwardly at their upper opening edges to form flared recesses 221. Inner edges of the flared recesses 221 are oblique so as to be closer to the side wall surfaces at the top and farther away from the side wall surfaces at the bottom.

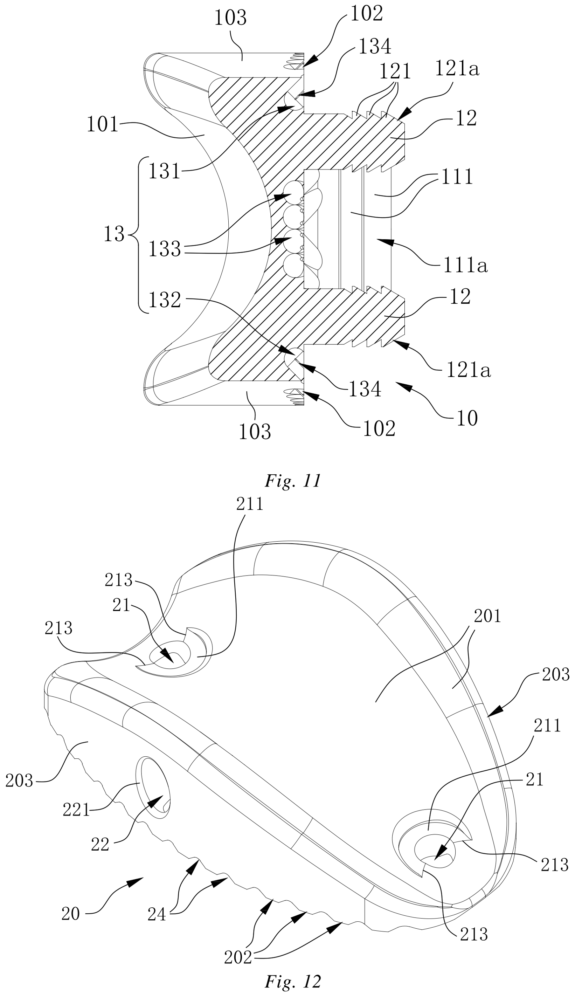

As shown in Figs. 12 and 14, the upper surface 201 of the trial implant is generally a smooth curved surface defining a valley-like profile in a widthwise direction thereof, which is sunken in the middle and raised at both ends, and a ridged profile in a lengthwise direction, which is swollen in the middle and slopes down towards both ends. Each locating hole 21 may define a boss 211 with a flat surface around its upper opening edge, which is configured for locating of a drill by bringing an end face of the drill into abutment against the flat surface. Since the upper surface 201 of the trial implant defines a ridged profile in the lengthwise direction, which is swollen in the middle and slopes down towards both ends, and because the locating holes 21 are located at the two ends of the trial implant 20 opposing each other in the lengthwise direction, the bosses 211 are defined around the upper opening edges of the locating holes 21 so that the boss surfaces are joined, on the side closer to the center of the trial implant 20, by vertical cylindrical surfaces 212, which are in turn joined by portions of the upper surface 201 of the trial implant above the boss surfaces, and on the side farther away from the center of the trial implant 20, by portions of the upper surface 201 of the trial implant below the boss surfaces at joint edges 213. Specifically, as shown in Fig. 16, the boss surfaces of the bosses 211 are generally C-shaped. Additionally, in order to improve orientation accuracy of the prosthesis 10 during its implantation, it is made sure that the cores of the holes drilled using the drilling shafts coincide with the cores of the locating holes 21. As shown in Fig. 17, the core lines of the locating holes 21 are perpendicular to the base surface 23, and the boss surfaces of the bosses 211 are perpendicular to the core lines of the locating holes 21.

## Claims

1. A biological patellar groove replacement assembly, **characterized in** comprising a prosthesis (10), a trial implant (20) and a base block (30),
an upper surface (101) of the prosthesis being a continuous curved surface defining a patellar groove, a lower surface (102) of the prosthesis being generally horizontally oriented, the lower surface (102) of the prosthesis provided thereon with a centrally arranged first mounting peg (11) and a second mounting peg (12) arranged near an outer peripheral edge of the prosthesis (10), cylindrical bodies of the first mounting peg (11) and the second mounting pegs (12) extending from the lower surface (102) of the prosthesis away from the upper surface (101) of the prosthesis,
an upper surface (201) of the trial implant having a contour generally coinciding with that of the upper surface (101) of the prosthesis, a lower surface (202) of the trial implant being generally horizontally oriented, the upper surface (201) of the trial implant and the lower surface (202) of the trial implant having a maximum height difference consistent with a maximum height difference of the upper surface (101) of the prosthesis and the lower surface (102) of the prosthesis, the trial implant (20) defining locating holes (21) extending through the upper surface (201) of the trial implant and the lower surface (202) of the trial implant and tuning holes (22) extending through side wall surfaces (203) of the trial implant and the lower surface (202) of the trial implant, cores of the locating holes (21) having orientations at the lower surface (202) of the trial implant coinciding with orientations of cores of the second mounting pegs (12) at the lower surface (102) of the prosthesis,
the base block (30) generally resembling a plate, the base block (30) defining a first locating hole (31) and second locating holes (32), all extending through an upper surface (301) of the base block and a lower surface (302) of the base block, a core of the first locating hole (31) having an orientation at the lower surface (302) of the base block coinciding with an orientation of a core of the first mounting peg (11) at the lower surface (102) of the prosthesis, cores of the second locating hole (32) having orientations at the lower surface (302) of the base block coinciding with orientations of cores of the second mounting pegs (12) at the lower surface (102) of the prosthesis.

2. The biological patellar groove replacement assembly according to claim 1, **characterized in that** the lower surface (102) of the prosthesis defines osseointegration holes (13) for growth of bone tissue therein, **in that** the lower surface (202) of the trial implant has an anti-slip texture, and **in that** each of the upper surface (301) of the base block and the lower surface (302) of the base block has an anti-slip texture.

3. The biological patellar groove replacement assembly according to claim 2, **characterized in that** outer peripheral contours of the lower surface (102) of the prosthesis, the lower surface (202) of the trial implant and the lower surface (302) of the base block coincide with one another, and **in that** the prosthesis (10), the trial implant (20) and the base block (30) are all axisymmetric, wherein each of the prosthesis (10), the trial implant (20) and the base block (30) has an axis of symmetry, which is parallel to a lengthwise direction of the lower surface of the component and is located at a center of the lower surface of the component in a widthwise direction thereof.

4. The biological patellar groove replacement assembly according to claim 2, **characterized in that** the osseointegration holes (13) are blind holes and are open obliquely outwards towards the bottom of the prosthesis (10),
wherein the osseointegration holes (13) include first holes (131) and second holes (132), core lines of the first holes (131) and the second holes (132) intersecting above the lower surface (102) of the prosthesis, or being non-coplanar straight lines, common perpendicular line segments of which are located above the lower surface (102) of the prosthesis; and
wherein the osseointegration holes (13) are scattered across the entire lower surface (102) of the prosthesis, in which the first holes (131) have parallel cores and form a first set of holes, and the second holes (132) have parallel cores and form a second set of holes, the first set of holes and the second set of holes located on opposite sides of the lower surface (102) of the prosthesis.

5. The biological patellar groove replacement assembly according to claim 4, **characterized in that**, when the second mounting pegs (12) are arranged in symmetry on the two sides of the lower surface (102) of the prosthesis, third holes (133) are provided in a region between two of the second mounting pegs (12), the plane containing core lines of the third holes (133) is perpendicular to planes containing the core lines of the first holes (131) or the second holes (132), the third holes (133) have parallel cores and form a third set of holes.

6. The biological patellar groove replacement assembly according to claim 4, **characterized in that** a notch (134) extends through adjacent walls of each adjacent pair of the osseointegration holes (13) with parallel cores, and **in that** the osseointegration holes (13) are arranged on a bottom portion of a side wall surface of the prosthesis (10), or at a joint of the side wall surface of the prosthesis (10) and the lower surface (102) of the prosthesis, and bottoms of the osseointegration holes (13) have a distance less than 2 mm from the lower surface (102) of the prosthesis.

7. The biological patellar groove replacement assembly according to claim 1, **characterized in that** the first mounting peg (11) has a greater outer peripheral contour than the second mounting pegs (12) and a size in a widthwise direction of the prosthesis (10), which is greater than 1/3 of a maximum size of the prosthesis (10) in the widthwise direction, and **in that** the second mounting pegs (12) are provided at front and rear ends of the prosthesis (10).

8. The biological patellar groove replacement assembly according to claim 7, **characterized in that** a depending end of the first mounting peg (11) defines a first retention collar (111) and **in that** depending ends of the second mounting pegs (12) define second retention collars (121), wherein:
a maximum distance between collar surfaces of the second retention collars (121) and the lower surface (102) of the prosthesis is greater than a maximum distance between a collar surface of the first retention collar (111) and the lower surface (102) of the prosthesis; or
the first retention collar (111) is arranged adjacent a depending end face of the first mounting peg (11) and has a first tapered collar surface (111a) joining the depending end face of the first mounting peg (11), the second retention collars (121) are arranged adjacent depending end faces of the second mounting pegs (12) and have second tapered collar surfaces (121a) joining the depending end faces of the second mounting pegs (12), and a degree of taper of the first tapered collar surface (111a) is greater than or equal to a degree of taper of the second tapered collar surfaces (121a); or
the cylindrical bodies of the second mounting pegs (12) have a projecting height greater than or equal to a projecting height of the cylindrical body of the first mounting peg (11).

9. The biological patellar groove replacement assembly according to claim 8, **characterized in that** the first mounting peg (11) and the second mounting pegs (12) are generally circular, wherein the cores of the first mounting peg (11) and the second mounting pegs (12) are perpendicular to the lower surface (102) of the prosthesis, and **in that**
the locating holes (21) and the second locating holes (32) each have a diameter smaller than or equal to a maximum outer diameter of the second mounting pegs (12), wherein the first locating hole (31) has a diameter smaller than or equal to a maximum outer diameter of the first mounting peg (11).

10. The biological patellar groove replacement assembly according to claim 2, **characterized in that** the upper surface (101) of the prosthesis is a smooth curved surface defining a valley-like profile in a widthwise direction of the prosthesis (10), which is sunken in the middle and raised at both ends, and a ridged profile in a lengthwise direction of the prosthesis (10), which is swollen in the middle and slopes down towards both ends, wherein the upper surface (201) of the trial implant comprises a curved surface portion coinciding with the upper surface (101) of the prosthesis; valleys of the anti-slip texture on the lower surface (202) of the trial implant are located on a single planar base surface (23); the core lines of the locating holes (21) are perpendicular to the base surface (23), and core lines of the tuning holes (22) form an acute angle γ with the base surface (23); and the anti-slip texture arranged on the trial implant (20) comprises trial implant grooves (24) extending generally in a widthwise direction of the trial implant (20).

11. An operation method of the biological patellar groove replacement assembly according to any one of claims 1 to 10, comprising the steps of:
A. with the lower surface (202) of the trial implant fitting against an osteotomy surface of a femur, applying pointed shafts through the tuning holes (22) and inserting them into the osteotomy surface of the femur, thereby preliminarily positioning the trial implant (20);
B. determining whether a lengthwise direction and tilt angle of a patellar groove of the upper surface (201) of the trial implant are consistent with a designed patellar track and, if so, proceeding to step C, or otherwise, removing the pointed shafts, tuning an orientation of the trial implant (20) and looping back to step A;
C. applying second drilling shafts through the locating holes (21) in the trial implant (20) and drilling second mounting holes in the osteotomy surface of the femur;
D. with the second drilling shafts being retained engaged with the osteotomy surface of the femur, pulling off the pointed shafts, separating the trial implant (20) from the osteotomy surface of the femur and then guiding it out along shaft bodies of the second drilling shafts;
E. with the second drilling shafts being inserted into the second locating holes (32) of the base block (30), guiding the base block (30) down along the shaft bodies of the second drilling shafts until it fits against the osteotomy surface of the femur, applying a first drilling shaft through the first locating hole (31) of the base block (30) and drilling a first mounting hole in the osteotomy surface of the femur;
F. separating the base plate (30) from the osteotomy surface of the femur, guiding it out along the shaft bodies of the first and second drilling shafts and removing the first and second drilling shafts; and
G. with the first mounting peg (11) of the prosthesis (10) abutting against an opening edge of the first mounting hole and the second mounting pegs (12) of the prosthesis (10) abutting against opening edges of the second mounting holes, securely press-fitting the prosthesis (10) onto the osteotomy surface of the femur so that the lower surface of the prosthesis (101) fits against the osteotomy surface of the femur, completing implantation of the prosthesis (10).
